# EUROPÄISCHE PATENTSCHRIFT

(11) **EP 3 256 242 B1**
(45) Veröffentlichungstag und Bekanntmachung des Hinweises auf die Patenterteilung: **25.03.2020**
(21) Anmeldenummer: 16726010.8
(22) Anmeldetag: 12.05.2016
(51) Int. Cl.: B01J 19/00

(54) **BEHÄLTERVORRICHTUNG FÜR CHEMISCHE UND BIOLOGISCHE PROZESSE**
CONTAINER DEVICE FOR CHEMICAL AND BIOLOGICAL PROCESSES
SYSTÈME DE CONTENANT POUR PROCÉDÉS CHIMIQUES ET BIOLOGIQUES

(30) Priorität: 06.07.2015 DE 102015008766
(43) Veröffentlichungstag der Anmeldung: 20.12.2017
(73) Patentinhaber: Sartorius Stedim Biotech GmbH, 37079 Göttingen (DE)
(72) Erfinder: SCHÄFER, Jan, 34295 Edermünde (DE); MANZKE, Christian, 37120 Bovenden (DE); EVRARD, Etienne, 1370 Lathuy (BE)
(74) Vertreter: Müller-Boré & Partner Patentanwälte PartG mbB
(86) Internationale Anmeldenummer: PCT/EP2016/000777
(87) Internationale Veröffentlichungsnummer: WO 2017/005335

(56) Entgegenhaltungen:
- WO-A1-00/70087
- WO-A2-2011/006032
- US-A- 5 536 471
- US-A1- 2005 278 066

## Beschreibung

Die vorliegende Erfindung betrifft eine Behältervorrichtung.

In der Industrie werden oftmals Behälteraufnahmen, sogenannte "Palletanks", verwendet, um darin Einwegbehälter aufzunehmen. Der Inhalt der Einwegbehälter kann in den Behälteraufnahmen gemischt, homogenisiert, transportiert (beispielsweise zum filtrieren), temperiert und/oder einfach gelagert werden. Auch können Inhalte den Einwegbehältern zugeführt oder von diesen abgeführt werden, während sich der Einwegbehälter in der Behälteraufnahme befindet, wobei der Inhalt des Einwegbehälters in der Behälteraufnahme gewogen werden kann. Des Weiteren ist es möglich, chemische und/oder biologische Prozesse in den Behälteraufnahmen durchzuführen.

Je nach durchzuführendem Prozess muss die Behälteraufnahme zu entsprechenden Bearbeitungsstationen bewegt werden, um die Behälteraufnahme bzw. den Einwegbehälter an entsprechende Vorrichtungen anzuschließen. Die Bearbeitungsstationen weisen üblicherweise jeweils eine Steuerungsvorrichtung zum Steuern der Vorrichtungen auf. Gegebenenfalls muss eine in der Behälteraufnahme und/oder in dem Einwegbehälter vorhandene Sensorik an die Steuerungsvorrichtung der entsprechenden Bearbeitungsstation angeschlossen werden.

Der Umgang mit den herkömmlichen Behälteraufnahmen ist sehr aufwendig, da für jeden durchzuführenden Prozess die Behälteraufnahme mit dem Einwegbehälter zu der entsprechenden Bearbeitungsstation bewegt und dort an die entsprechenden Vorrichtungen angeschlossen werden muss. Des Weiteren muss an jeder Bearbeitungsstation die entsprechende Steuerungsvorrichtung für den
durchzuführenden Prozess konfiguriert werden. WO2011006032 offenbart eine modulare Vorrichtung mit einem Behälter.

Es ist daher Aufgabe der vorliegenden Erfindung, eine Behältervorrichtung bereitzustellen, welche einfacher in der Handhabung ist.

Diese Aufgabe wird durch den Gegenstand des unabhängigen Anspruchs gelöst. Bevorzugte Ausführungsformen ergeben sich aus den abhängigen Ansprüchen.

Gemäß der vorliegenden Erfindung wird eine Behältervorrichtung gemäß Anspruch 1 bereitgestellt. Vorteilhafterweise ermöglicht die erfindungsgemäße Behältervorrichtung die Integration der für die typischen durchzuführenden Prozesse notwendigen Vorrichtungen zu einer kompakten Prozess-Einheit. Hierdurch wird die Handhabung der Behältervorrichtung stark vereinfacht, da die Notwendigkeit des Anschließens der Behältervorrichtung an verschiedene Vorrichtungen an verschiedenen Bearbeitungsstationen reduziert werden kann oder sogar komplett entfallen kann. Auch wenn die Behältervorrichtung zu einer Bearbeitungsstation bewegt werden muss, geschieht dies auf einfache Weise als Prozess-Einheit, so dass lediglich die fehlenden Vorrichtungen bzw. Anschlüsse an die Behältervorrichtung angeschlossen werden müssen. Hierbei ist ferner vorteilhaft, dass die für einen Behälter durchzuführenden Prozesse mittels einer einzigen Steuerungsvorrichtung durchgeführt werden können, welche ständig Teil der Prozess-Einheit ist. Dies reduziert nicht nur den Konfigurationsaufwand der Steuerungsvorrichtung, sondern verbessert auch die Überwachungsmöglichkeiten der Prozesse.

Die Behälteraufnahme kann im Wesentlichen quaderförmig oder würfelförmig ausgebildet sein. Die Behälteraufnahme kann beispielsweise einen im Wesentlichen wannenförmigen Hauptkörper und einen im Wesentlichen plattenförmigen Deckel aufweisen. Die Behälteraufnahme kann doppelwandig ausgeführt sein, wobei der Zwischenraum zwischen der Innenwandung und der Außenwandung von einem Temperiermittel durchströmbar ausgebildet sein kann und/oder Temperiermittelleitungen aufweisen kann, um einen Aufnahmeraum der Behälteraufnahme zu temperieren. In der Behälteraufnahme, insbesondere in dem Aufnahmeraum davon, kann ein Behälter zumindest teilweise aufgenommen werden, oder es können eine Mehrzahl von Behältern zumindest teilweise aufgenommen werden. Die Behälteraufnahme hat eine oder mehrere Öffnungen in einer oder mehreren der Seitenwände und/oder im Boden und/oder im Deckel, um eine oder mehrere mit dem Behälter verbundene Schläuche, insbesondere Fluidleitungen, nach Außen durchzuführen. Die Behälteraufnahme kann beispielsweise ein Aufnahmevolumen bzw. Fassungsvolumen von etwa 10 bis 3000 Litern, vorzugsweise von etwa 50 bis 500 Litern, aufweisen. Die Behälteraufnahme kann beispielsweise ein "Palletank" sein.

Bei dem Behälter kann es sich um einen Einwegbehälter, insbesondere um einen Einweg-Kunststoffbeutel, handeln. Der Behälter kann beispielsweise ein Bioreaktorbeutel sein. Die Erfindung ist jedoch nicht auf Einweg-Kunststoffbeutel beschränkt. Der Behälter kann einen oder mehrere Anschlüsse aufweisen, wobei es sich um Anschlüsse zum Befüllen, zum Entleeren, zum Begasen und/oder zum Entgasen handeln kann. Über die Anschlüsse können Schläuche bzw. Fluidleitungen mit dem Behälter verbunden sein. Bei den Fluidleitungen kann es sich insbesondere um Kunststoffschläuche, vorzugsweise Einweg-Kunststoffschläuche, handeln. Der Behälter kann einen oder mehrere integrierte Sensoren aufweisen, wie beispielsweise Temperatur-, Druck-, pH-, Leitfähigkeits- und/oder Sauerstoffsensoren, welche mit der Steuerungsvorrichtung verbindbar sind, beispielsweise über Signalleitungen, ein Bussystem und/oder über Funk (Bluetooth, WLAN, etc.). Der Behälter kann ferner ein integriertes Rührwerk, Zugabeports und/oder Probeentnahmesysteme aufweisen. Der Inhalt des Behälters kann in der Behälteraufnahme gemischt, homogenisiert, filtriert, temperiert und/oder einfach gelagert/transportiert werden.

Das Ventil dient zur Steuerung eines Zu- und/oder Abflusses des Fluids in der Fluidleitung zum und/oder vom Behälter. Das zu- oder abfließende Fluid kann auch Feststoffe enthalten. Bei dem Ventil kann es sich um ein berührungsloses Ventil, insbesondere um ein Klemmventil, handeln. Bei einem Klemmventil wird die Fluidleitung bei Betätigung durch das Ventil eingeklemmt, um den Fluidstrom durch die Fluidleitung zu verringern oder zu unterbrechen. Das Klemmventil kann für Schäuche von 1/4" bis 1" ausgelegt sein, vorzugsweise für 3/8"-Schläuche oder 1/2"-Schläuche ausgelegt sein. Es können jedoch auch Klemmventile für sowohl 3/8"-Schläuche als auch 1/2"-Schläuche vorgesehen sein. Die Betätigung kann magnetisch, pneumatisch und/oder hydraulisch erfolgen. Bei dem Ventil kann es sich jedoch auch um ein berührendes Ventil, beispielsweise ein Einweg-Membranventil, handeln. Die Behältervorrichtung kann eine Vielzahl von Ventilen aufweisen, beispielsweise 2 bis 20 Ventile aufweisen.

Die Pumpvorrichtung dient der Erzeugung des Förderdrucks des Fluids in der Fluidleitung, wobei die Förder- bzw. Pumprichtung zum Behälter hin oder vom Behälter weg gerichtet sein kann. Mittels der Pumpvorrichtung kann eine Rezirkulation des Fluides zur Durchmischung des Behälters ermöglicht werden. Die Pumpvorrichtung muss nicht zwingend mit derselben Fluidleitung gekoppelt sein, welche mit dem Ventil gekoppelt ist. Es kann eine Fluidleitung auch lediglich mit der Pumpvorrichtung gekoppelt sein oder lediglich mit dem Ventil gekoppelt sein. Es können auch zwei Fluidleitungen vorgesehen sein, welche jeweils mit der Pumpvorrichtung oder dem Ventil gekoppelt sind. Die Pumpvorrichtung kann eine berührungslose Pumpe, wie beispielsweise eine peristaltische Pumpe, in welche die Fluidleitung eingesetzt wird, aufweisen. Die Pumpvorrichtung kann jedoch auch eine berührende Pumpe, beispielsweise eine Einweg-Membranpumpe, aufweisen. Die Pumpvorrichtung kann eine Vielzahl von Pumpen aufweisen, mittels welcher Förderdrücke in einer Vielzahl von Fluidleitungen erzeugt werden kann. Vorzugsweise weist die Pumpvorrichtung zwei Pumpen, insbesondere zwei peristaltische Pumpen auf, wobei eine der Pumpen der Fluidzufuhr, der Fluidabfuhr und/oder der Fluidzirkulation dient, und die andere Pumpe für Titrationsprozesse verwendbar ist. Hierbei können die zwei Pumpen für verschieden hohe Durchsätze ausgelegt sein.

Die Steuerungsvorrichtung kann über Signalleitungen, ein Bussystem und/oder über Funk (Bluetooth, WLAN, etc.) mit dem Ventil und/oder der Pumpvorrichtung verbunden sein. Die Steuerungsvorrichtung kann mit sämtlichen für den durchzuführenden Prozess nötigen Vorrichtungen und Sensoren in Verbindung bringbar sein, vorzugsweise über Signalleitungen, ein Bussystem und/oder über Funk (Bluetooth, WLAN, etc.). Die Steuerungsvorrichtung kann ausgelegt sein, als I/O-Box (Input/Output-Box) mit einer externen Regelungsvorrichtung, beispielsweise einem Rechner, zu kommunizieren, wobei die Kommunikation über Signalleitungen, ein Bussystem oder auch drahtlos erfolgen kann. Die Steuerungsvorrichtung kann I/O-Module, Bus-Schnittstellen, eine Energie-Versorgung und/oder eine Not-Aus-Einrichtung aufweisen. Die Steuerungsvorrichtung kann die mit ihr verbundenen Vorrichtungen, wie das Ventil und/oder die Pumpvorrichtung, ansteuern. Die Steuerungsvorrichtung kann auch ausgelegt sein, die durchzuführenden Prozesse zu steuern, zu regeln und/oder zu dokumentieren. Die durchzuführenden Prozesse können Mischungsprozesse, Filtrationsprozesse, Temperierungsprozesse, Lagerprozesse, Titrationsprozesse, chemische Reaktionsprozesse, biologische Reaktionsprozesse, Befüllungsprozesse, Entleerungsprozesse, Begasungsprozesse und/oder Entgasungsprozesse umfassen.

Der Rahmen kann eine Schweißkonstruktion aus Stahl- und/oder Aluminiumprofilen, beispielsweise Vierkantrohren, sein. Der Rahmen kann im Wesentlichen quaderförmig ausgebildet sein und vier außenliegende, im Wesentlichen vertikale Säulen aufweisen, welche durch Längsträger und/oder -platten und Querträger und/oder -platten verbunden sind. Die Behälteraufnahme ist vorzugsweise im Wesentlichen mittig in dem Rahmen angeordnet und mit diesem verbunden. Die Behälteraufnahme kann derart in bzw. an dem Rahmen angeordnet sein, dass die Auflagefläche des Behälters in der Behälteraufnahme auf einer Höhe von etwa 50 cm bis etwa 100 cm liegt, was die Handhabung der Behältervorrichtung weiter vereinfacht. Die Behälteraufnahme, das Ventil, die Pumpvorrichtung und die Steuerungsvorrichtung sind vorzugsweise jeweils für sich mit dem Rahmen verbunden bzw. an dieser befestigt. Die Befestigung kann beispielsweise mittels Verschraubung und/oder Verschweißung erfolgen. Die Behälteraufnahme kann auf Schienen auf dem Rahmen gelagert sein, mittels welcher die Behälteraufnahme relativ zum Rahmen verlagert werden kann, um das Be- und Entladen des Behälters in bzw. von der Behälteraufnahme zu vereinfachen.

Vorzugsweise kann die Behältervorrichtung weiter eine Wägevorrichtung aufweisen, wobei der Rahmen die Wägevorrichtung trägt.

Die Wägevorrichtung dient dem Wiegen des Inhalts des Behälters. Die Wägevorrichtung ist vorzugsweise ebenfalls mit der Steuerungsvorrichtung verbunden, vorzugsweise über eine Signalleitung, ein Bussystem und/oder über Funk (Bluetooth, WLAN, etc.). Die Information über die Masse des Inhalts des Behälters kann zur Regelung von Prozessen herangezogen werden. Vorzugsweise werden vor dem Start des Prozesses das Gewicht des Behälters und der Fluidleitungen austariert. Alternativ oder zusätzlich kann die Behältervorrichtung einen Füllstandssensor aufweisen, welcher das Volumen des Inhalts des Behälters erfasst, und welcher mit der Steuerungsvorrichtung verbunden ist.

Vorzugsweise weist die Wägevorrichtung zumindest eine Wägezelle auf, welche als Auflager der Behälteraufnahme zwischen der Behälteraufnahme und dem Rahmen angeordnet ist.

Die Behälteraufnahme ist insbesondere ausschließlich über die Wägezelle mit dem Rahmen verbunden. Vorzugsweise weist die Wägevorrichtung drei Wägezellen auf, welche vorzugsweise in gleichmäßigen Abständen an der Unterseite der Behälteraufnahme zwischen der Behälteraufnahme und dem Rahmen angeordnet sind. Die Wägezellen können jeweils mit der Steuerungsvorrichtung verbunden sein, beispielsweise über Signalleitungen, ein Bussystem und/oder über Funk (Bluetooth, WLAN, etc.). Das Gewicht der Behälteraufnahme sowie ggf. des Temperiermittels kann austariert werden.

Vorzugsweise weist der Rahmen an seiner Unterseite Räder und/oder Rollen auf, mittels welcher die Behältervorrichtung als Einheit bewegt werden kann.

Die Räder bzw. Rollen tragen das Gesamtgewicht der Behältervorrichtung. Durch die Räder bzw. Rollen wird das Hin- und Herbewegen der Behältervorrichtung und somit die Handhabung weiter vereinfacht. Der Rahmen kann insbesondere vier Räder aufweisen. Die Räder sind vorzugsweise zu einer vertikalen Achse schwenkbar an dem Rahmen angeordnet.

Vorzugsweise weist die Behälteraufnahme einen Temperiermittel-Zulauf und einen Temperiermittel-Ablauf auf, welche mit einer Temperier-Regelungsvorrichtung verbindbar sind, um den in der Behälteraufnahme aufgenommenen Behälter zu temperieren.

Die Behälteraufnahme kann doppelwandig ausgeführt sein, wobei der Zwischenraum zwischen der Innenwandung und der Außenwandung von einem Temperiermittel durchströmbar ausgebildet sein kann und/oder Temperiermittelleitungen aufweisen kann, um einen Aufnahmeraum der Behälteraufnahme zu temperieren. Das Temperiermittel wird über den Temperiermittel-Zulauf zugeführt und über den Temperiermittel-Ablauf abgeführt. Temperiermittel-Zulauf und -Ablauf können Kupplungen zur vereinfachten Verbindung mit den Rohren bzw. Schläuchen der Temperiermittel-Regelungsvorrichtung aufweisen. Die Temperiermittel-Regelungsvorrichtung kann eine Zirkulationspumpe für den Temperiermittel-Kreislauf aufweisen, sowie einen Temperieraktor zum Kühlen und/oder Heizen sowie entsprechende Sensoren aufweisen. Die Temperiermittel-Regelungsvorrichtung kann mit der Steuerungsvorrichtung verbindbar sein, beispielsweise über eine Signalleitung, ein Bussystem und/oder über Funk (Bluetooth, WLAN, etc.), und kann von dieser angesteuert bzw. geregelt werden. Die Temperiermittel-Regelungsvorrichtung kann ebenfalls an dem Rahmen angeordnet werden und Teil der Behältervorrichtung darstellen. Der Temperiermittel-Zulauf und/oder der Temperiermittel-Ablauf können auch mit der Steuerungsvorrichtung verbunden sein.

Vorzugsweise weist die Behältervorrichtung eine Rührvorrichtung auf, welche an dem Rahmen und unter der Behälteraufnahme angeordnet ist.

Die Rührvorrichtung kann ebenfalls mit der Steuerungsvorrichtung verbindbar sein, beispielsweise über eine Signalleitung, ein Bussystem und/oder über Funk (Bluetooth, WLAN, etc.), und kann von dieser angesteuert bzw. geregelt werden. Die Rührvorrichtung wird ebenfalls von dem Rahmen getragen. Bei der Rührvorrichtung handelt es sich vorzugsweise um einen Magnetrührer, bei dem ein im Behälter angeordnetes Rührelement magnetisch in Rotation versetzt wird.

Vorzugsweise ist das Ventil ein Klemmventil, und/oder die Pumpvorrichtung weist eine peristaltische Pumpe auf.

Vorzugsweise weist die Behälteraufnahme eine Durchführöffnung zum Durchführen der Fluidleitung auf, wobei das Ventil im Bereich der Durchführöffnung an dem Rahmen angeordnet ist.

Die Durchführöffnung kann ausgebildet sein, eine Vielzahl von Fluidleitungen durch die Wandung der Behälteraufnahme durchzuführen. Vorzugsweise weist die Behälteraufnahme an einer Seitenwand eine Durchführöffnung auf, wobei in dem Bereich der Durchführöffnung zumindest ein Ventil an dem Rahmen, insbesondere an einer vertikalen Säule davon, angeordnet ist.

Vorzugsweise weist die Steuerungsvorrichtung ein Bussystem zur Kommunikation mit einer externen Vorrichtung auf.

Bei der externen Vorrichtung kann es sich um einen externen Rechner handeln, wobei die Kommunikation über eine Signalleitung, ein Bussystem oder drahtlos erfolgen kann. Mittels des Rechners kann der durchzuführende Prozess auf einfache Weise konfiguriert, gesteuert bzw. geregelt, dokumentiert und/oder ausgewertet werden.

Vorzugsweise weist die Steuerungsvorrichtung eine Eingabevorrichtung auf.

Die Eingabevorrichtung kann beispielsweise ein Eingabe-Panel sein. Die Steuerungsvorrichtung kann ferner eine Anzeige, beispielsweise einen Monitor bzw. ein Display, aufweisen. Die Steuerungsvorrichtung kann auch ein Touchpad aufweisen, welcher eine Eingabevorrichtung und eine Anzeige vereint. Mittels der Eingabevorrichtung können Prozesse ohne die Verwendung einer externen Vorrichtung durchgeführt werden.

Im Folgenden wird eine Ausführungsform der Erfindung anhand der beigefügten Figuren näher beschrieben.

Es zeigen:
- **Fig. 1**: eine perspektivische Ansicht einer Behältervorrichtung gemäß einer Ausführungsform;
- **Fig. 2**: eine weitere perspektivische Ansicht der Behältervorrichtung gemäß der Ausführungsform;
- **Fig. 3**: eine Seitenansicht der Behältervorrichtung gemäß der Ausführungsform;
- **Fig. 4**: eine weitere Seitenansicht der Behältervorrichtung gemäß der Ausführungsform.

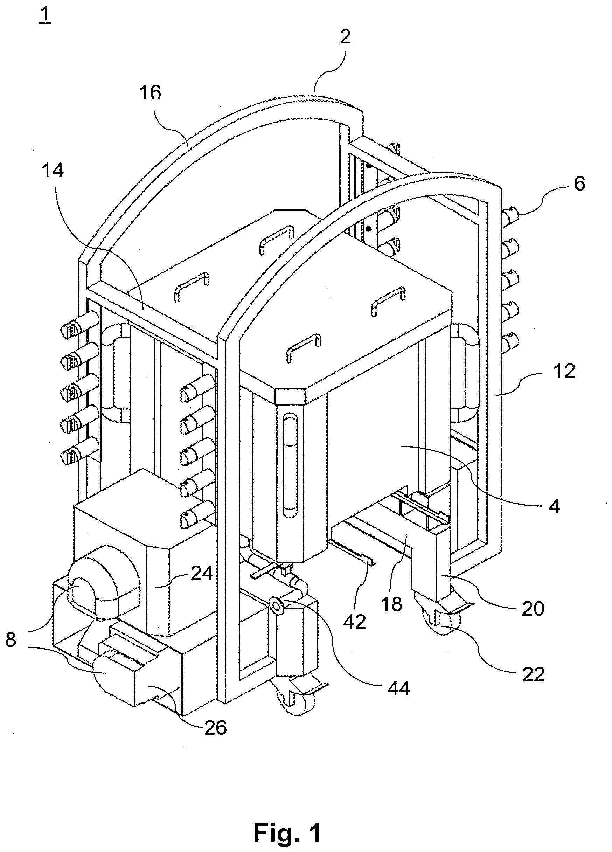

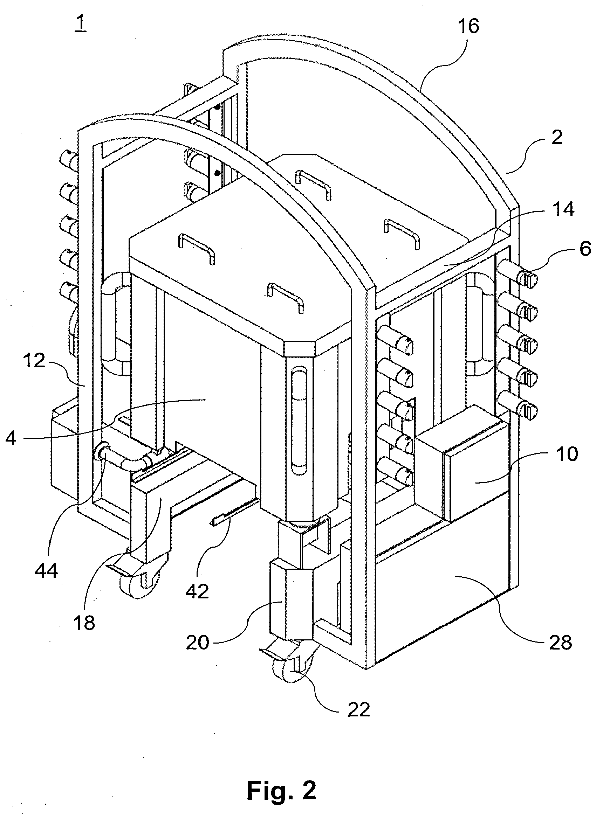

Fig. 1 und Fig. 2 zeigen jeweils perspektivische Ansichten einer Behältervorrichtung 1 gemäß einer Ausführungsform aus verschiedenen Blickwinkeln. Die Behältervorrichtung 1 weist einen Rahmen 2 auf, an welchem eine Behälteraufnahme 4, eine Vielzahl von Ventilen 6, eine Pumpvorrichtung 8 und eine Steuerungsvorrichtung 10 angeordnet sind. Der Rahmen 2 trägt die Behälteraufnahme 4, die Ventile 6, die Pumpvorrichtung 8 und die Steuerungsvorrichtung 10.

Der Rahmen 2 gemäß der Ausführungsform ist im Wesentlichen eine Schweißkonstruktion aus Vierkantrohren und weist vier außenliegende, im Wesentlichen vertikale Säulen 12 auf, welche durch im Wesentlichen horizontale Querträger 14 und Längsträger 16 verbunden sind. Der Rahmen 2 weist ferner mittig einen Gestellabschnitt 18 auf, welcher insbesondere als Auflager für die Behälteraufnahme 4 dient. Die vier im Wesentlichen vertikalen Gestellbeine 20 weisen an ihren unteren Enden jeweils schwenkbar gelagerte Räder 22 auf. An den Rädern 22 kann jeweils eine Feststellbremse angeordnet sein, vorzugsweise mindestens an jeweils zwei der Räder 22. Die Räder 22 tragen das Gewicht der Behältervorrichtung 1 und erlauben ein einfaches Bewegen der Behältervorrichtung 1. Der Rahmen 2 bzw. die Behältervorrichtung 1 weist eine schmale Seite und eine breite Seite auf.

Die Pumpvorrichtung 8 weist eine erste peristaltische Pumpe 24 und eine zweite peristaltische Pumpe 26 auf. Die erste peristaltische Pumpe 24 kann beispielsweise zum Befüllen oder Entleeren des Behälters (nicht gezeigt) oder zur Rezirkulation des Fluids im Behälter verwendet werden. Die zweite peristaltische Pumpe 26 kann beispielsweise zur Titration, vorzugsweise in Kombination mit den Ventilen 6, verwendet werden. Sowohl die erste peristaltische Pumpe 24 als auch die zweite peristaltische Pumpe 26 sind mit der Steuerungsvorrichtung 10 mittels Signalleitungen (nicht gezeigt) verbunden und werden von dieser angesteuert. Die Pumpvorrichtung 8 ist zwischen zwei Säulen der schmalen Seite des Rahmens 2 an einem unteren, seitlichen Abschnitt des Rahmens 2 angeordnet.

Die Ventile 6 werden von der Steuerungsvorrichtung 10 angesteuert. Hierzu ist die Steuerungsvorrichtung 10 mit dem Ventil-Steuerungskasten 28 der Ventile 6 über eine Signalleitung (nicht gezeigt) verbunden. Die Behältervorrichtung 1 weist zwanzig Ventile 6 auf, welche jeweils in Gruppen von fünf an den Säulen 12 des Rahmens 2 im Wesentlichen auf der Höhe der Behälteraufnahme 4 angeordnet sind. Die Ventile 6 sind als Klemmventile ausgebildet und weisen jeweils eine Aufnahmerille für die Fluidleitungen (nicht gezeigt) des Behälters (nicht gezeigt) auf. Die Steuerungsvorrichtung 10 und der Ventil-Steuerungskasten 28 sind gegenüberliegend zur Pumpvorrichtung 8 zwischen zwei Säulen der schmalen Seite des Rahmens 2 an einem unteren, seitlichen Abschnitt des Rahmens 2 angeordnet. Die Steuerungsvorrichtung 10 und die Pumpvorrichtung 8 sind also an zwei gegenüberliegenden Seiten des Rahmens 2 angeordnet, wobei die Behälteraufnahme 4 im Wesentlichen mittig zwischen der Steuerungsvorrichtung 10 und der Pumpvorrichtung 8 angeordnet ist.

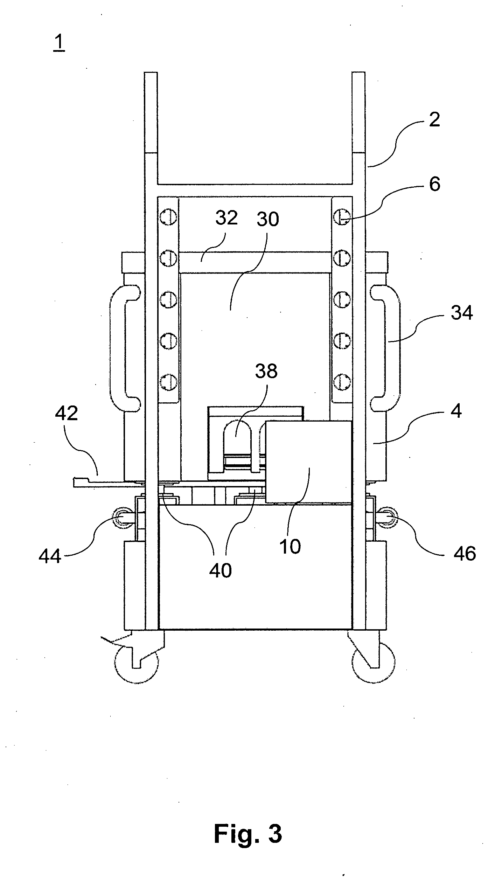

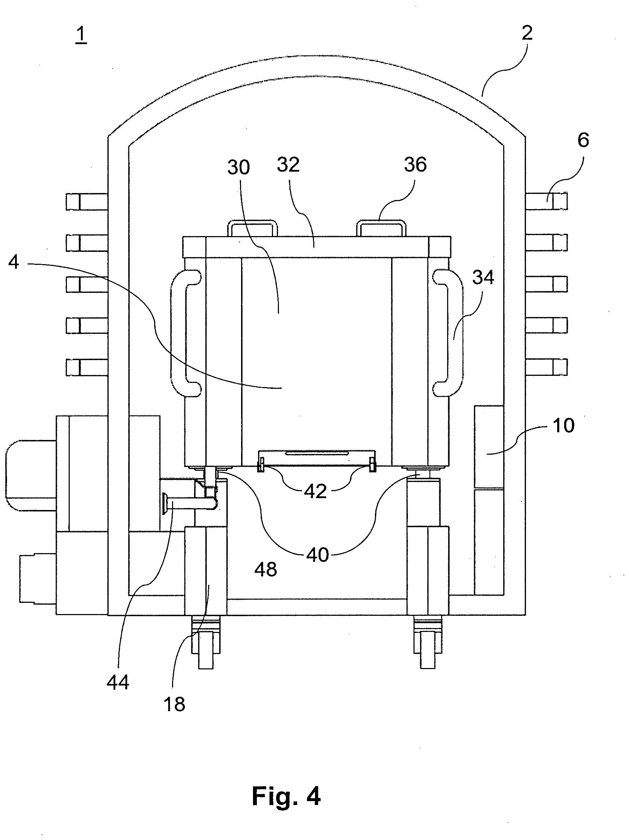

Fig. 3 zeigt eine Seitenansicht der schmalen Seite der Behältervorrichtung 1, und Fig. 4 zeigt eine Seitenansicht der breiten Seite der Behältervorrichtung 1. Die Behälteraufnahme 4 ist im Wesentlichen quaderförmig oder würfelförmig ausgebildet und weist einen im Wesentlichen wannenförmigen Hauptkörper 30 und einen im Wesentlichen plattenförmigen Deckel 32 auf. Die Form der Behälteraufnahme 4 ist jedoch nicht hierauf beschränkt und kann auch beispielsweise zylindrisch ausgebildet sein. Für ein vereinfachtes Bewegen der Behälteraufnahme 4 weist der Hauptkörper 30 an den vier äußeren Ecken davon Handhabungsgriffe 34 auf. Für ein vereinfachtes Abheben des Deckels 32 weist der Deckel 32 vier Deckelgriffe 36 auf.

Die Behälteraufnahme 4 weist an einer Seitenwand des Hauptkörpers 30 eine zur schmalen Seite der Behältervorrichtung 1 hin offene Durchführöffnung 38 auf. Durch die Durchführöffnung 38 können Fluidleitungen, welche mit dem Behälter verbunden sind, von Außen ins Innere der Behälteraufnahme 4 geführt werden. Die Durchführöffnung 38 ist derart dimensioniert, dass eine mögliche Temperierfunktion der Behälteraufnahme 4 nicht beeinträchtigt wird. Die Durchführöffnung 38 ist vorzugsweise groß genug, dass zumindest zwanzig Fluidleitungen, entsprechend der Anzahl der Ventile 6, hindurch geführt werden können.

Die Behälteraufnahme 4 ist über drei Wägezellen 40 mit dem Rahmen 2, insbesondere dem Gestellabschnitt 18 davon, verbunden. Die Wägezellen 40 stellen die Auflager für die Behälteraufnahme 4 dar. Die Wägezellen 40 sind mit der Steuerungsvorrichtung 10 über Signalleitungen verbunden. Die Behälteraufnahme 4 weist ferner ein Schienensystem 42 auf, mittels welcher die Behälteraufnahme 4 relativ zum Rahmen 2 verlagert werden kann, insbesondere seitlich von einem zentralen Bereich des Rahmens 2 herausgeschoben werden kann. Hierdurch wird das Be- und Entladen der Behälteraufnahme 4 vereinfacht.

Die Behälteraufnahme 4 bzw. der Hauptkörper 30 davon ist doppelwandig ausgebildet. In dem Zwischenraum zwischen der inneren Wandung und der äußeren Wandung der Behälteraufnahme 4 kann Temperiermittel bzw. können Temperiermittel-Leitungen aufgenommen werden. Bei der vorliegenden Ausführungsform ist eine Temperiermittel-Regelungsvorrichtung (nicht gezeigt) nicht Teil der Behältervorrichtung 1. Daher weist die Behälteraufnahme 4 lediglich einen Temperiermittel-Zulauf 44 und einen Temperiermittel-Ablauf 46 auf, welche an der Unterseite der Behälteraufnahme 4 angeordnet sind. Über den Temperiermittel-Zulauf 44 und den Temperiermittel-Ablauf 46 kann eine externe Temperiermittel-Regelungsvorrichtung angeschlossen werden, mittels welcher der Innenraum der Behälteraufnahme 4 temperiert werden kann. Die externe Temperiermittel-Regelungsvorrichtung kann mit der Steuerungsvorrichtung 10 über eine Signalleitung verbunden werden und von dieser angesteuert werden.

Bei der vorliegenden Ausführungsform ist des Weiteren eine Rührvorrichtung (nicht gezeigt) nicht Teil der Behältervorrichtung 1. Daher weist der Rahmen 2 bzw. der Gestellabschnitt 18 davon einen Freiraum 48 unterhalb der Behälteraufnahme 4 auf, so dass eine externe Rührvorrichtung, insbesondere ein Magnetrührer, von der Seite unter die Behälteraufnahme 4 geschoben werden kann, um den Inhalt des Behälters zu verrühren. Die externe Rührvorrichtung kann mit der Steuerungsvorrichtung 10 über eine Signalleitung verbunden werden und von dieser angesteuert werden.

### Bezugszeichenliste

- 1: Behältervorrichtung
- 2: Rahmen
- 4: Behälteraufnahme
- 6: Ventil
- 8: Pumpvorrichtung
- 10: Steuervorrichtung
- 12: Säule
- 14: Querträger
- 16: Längsträger
- 18: Gestellabschnitt
- 20: Gestellbein
- 22: Rad
- 24: erste peristaltische Pumpe
- 26: zweite peristaltische Pumpe
- 28: Ventil-Steuerungskasten
- 30: Hauptkörper
- 32: Deckel
- 34: Handhabungsgriff
- 36: Deckelgriff
- 38: Durchführöffnung
- 40: Wägezellen
- 42: Schienensystem
- 44: Temperiermittel-Zulauf
- 46: Temperiermittel-Ablauf
- 48: Freiraum

## Patentansprüche

1. Behältervorrichtung (1), aufweisend:
eine Behälteraufnahme (4) zur Aufnahme eines Behälters, wobei in einem Aufnahmeraum der Behälteraufnahme (4) zumindest ein Einwegbehälter aufnehmbar ist, wobei die Behälteraufnahme (4) zumindest eine Durchführöffnung (38) in einer oder mehreren Seitenwänden und/oder im Boden und/oder im Deckel (32) aufweist, um eine oder mehrere mit dem Einwegbehälter verbindbaren Fluidleitungen nach Außen durchzuführen;
zumindest ein Ventil (6) zur Steuerung eines Fluidstroms in einer mit dem Behälter verbundenen Fluidleitung;
eine Pumpvorrichtung (8) zur Erzeugung eines Förderdrucks des Fluids in der Fluidleitung;
eine Steuerungsvorrichtung (10), welche mit dem Ventil (6) und/oder der Pumpvorrichtung (8) in Verbindung bringbar ist, um das Ventil (6) und/oder die Pumpvorrichtung (8) zu steuern; und
einen Rahmen (2), welcher die Behälteraufnahme (4), das Ventil (6), die Pumpvorrichtung (8) und die Steuervorrichtung (10) trägt.

2. Behältervorrichtung (1) nach Anspruch 1, weiter aufweisend eine Wägevorrichtung (40), wobei der Rahmen (2) die Wägevorrichtung (40) trägt.

3. Behältervorrichtung (1) nach Anspruch 2, wobei die Wägevorrichtung (40) zumindest eine Wägezelle (40) aufweist, welche als Auflager der Behälteraufnahme (4) zwischen der Behälteraufnahme (4) und dem Rahmen (2) angeordnet ist.

4. Behältervorrichtung (1) nach einem der vorangehenden Ansprüche, wobei der Rahmen (2) an seiner Unterseite Räder (22) und/oder Rollen aufweist, mittels welcher die Behältervorrichtung (1) als Einheit bewegt werden kann.

5. Behältervorrichtung (1) nach einem der vorangehenden Ansprüche, wobei die Behälteraufnahme (4) einen Temperiermittel-Zulauf (44) und einen Temperiermittel-Ablauf (46) aufweist, welche mit einer Temperier-Regelungsvorrichtung verbindbar sind, um den in der Behälteraufnahme (4) aufgenommenen Behälter zu temperieren.

6. Behältervorrichtung (1) nach einem der vorangehenden Ansprüche, wobei die Behältervorrichtung (1) eine Rührvorrichtung aufweist, welche an dem Rahmen (2) und unter der Behälteraufnahme (4) angeordnet ist.

7. Behältervorrichtung (1) nach einem der vorangehenden Ansprüche, wobei das Ventil (6) ein Klemmventil ist, und/oder wobei die Pumpvorrichtung (8) eine peristaltische Pumpe (24, 26) aufweist.

8. Behältervorrichtung (1) nach einem der vorangehenden Ansprüche, wobei die Behälteraufnahme (4) eine Durchführöffnung (38) zum Durchführen der Fluidleitung aufweist, wobei das Ventil (6) im Bereich der Durchführöffnung (38) an dem Rahmen (2) angeordnet ist.

9. Behältervorrichtung (1) nach einem der vorangehenden Ansprüche, wobei die Steuervorrichtung (10) ein Bussystem zur Kommunikation mit einer externen Vorrichtung aufweist.

10. Behältervorrichtung (1) nach einem der vorangehenden Ansprüche, wobei die Steuervorrichtung (10) eine Eingabevorrichtung aufweist.

## Claims

1. Container device (1) comprising:
a container receiver (4) for receiving containers, wherein at least one single-use container is receivable in a receiving space of the container receiver (4), wherein the container receiver (4) has at least a feed-through aperture (38) in one or more side walls and/or the floor and/or the lid (32) in order to feed through to the outside one or more fluid conduits connectable with the single-use container;
at least one valve (6) for controlling a fluid stream in a fluid conduit connected with the container;
a pump device (8) for generating a feed pressure of the fluid in the fluid conduit;
a control device (10) connectable with the valve (6) and/or the pump device (8) in order to control the valve (6) and/or the pump device (8); and
a frame (2) supporting the container receiver (4), the valve (6), the pump device (8) and the control device (10).

2. Container device (1) according to claim 1, further having a weighing device (40), wherein the frame (2) supports the weighing device (40).

3. Container device (1) according to claim 2, the weighing device (40) having at least one load cell (40), which is arranged as a support of the container receiver (4) between the container receiver (4) and the frame (2).

4. Container device (1) according to any of the preceding claims, the frame (2) having wheels (22) and/or rollers on its underside, with which the container device (1) can be moved as a unit.

5. Container device (1) according to any of the preceding claims, the container receiver (4) having a temperature control means inlet (44) and a temperature control means outlet (46), which are connectable with a temperature control device, so as to control the temperature of the container received in the container receiver (4).

6. Container device (1) according to any of the preceding claims, the container device (1) having a stirring device, which is disposed on the frame (2) and under the container receiver (4).

7. Container device (1) according to any of the preceding claims, wherein the valve (6) is a pinch valve, and/or wherein the pump device (8) has a peristaltic pump (24, 26).

8. Container device (1) according to any of the preceding claims, the container receiver (4) having a feed-through aperture (38) for feeding through the fluid conduit, wherein the valve (6) is arranged on the frame (2) in the area of the feed-through aperture (38).

9. Container device (1) according to any of the preceding claims, the control device (10) having a bus system for communication with an external device.

10. Container device (1) according to any of the preceding claims, the control device (10) having an input device.

## Revendications

1. Dispositif de récipient (1) présentant :
une réception de récipient (4) pour la réception d'un récipient, dans lequel au moins un récipient à usage unique peut être reçu dans un espace de réception de la réception de récipient (4), dans lequel la réception de récipient (4) présente au moins une ouverture de passage (38) dans une ou plusieurs parois latérales et/ou dans le fond et/ou dans le couvercle (32) pour faire passer vers l'extérieur une ou plusieurs conduites de fluide pouvant être connectées au récipient à usage unique ;
au moins une soupape (6) pour la commande d'un courant de fluide dans une conduite de fluide connectée au récipient ;
un dispositif de pompe (8) pour la génération d'une pression de transfert du fluide dans la conduite de fluide ;
un dispositif de commande (10) qui peut être amené en connexion avec la soupape (6) et/ou le dispositif de pompe (8) pour commander la soupape (6) et/ou le dispositif de pompe (8) ; et
un cadre (2) qui porte la réception de récipient (4), la soupape (6), le dispositif de pompe (8) et le dispositif de commande (10).

2. Dispositif de récipient (1) selon la revendication 1, présentant en outre un dispositif de pesée (40), dans lequel le cadre (2) porte le dispositif de pesée (40).

3. Dispositif de récipient (1) selon la revendication 2, dans lequel le dispositif de pesée (40) présente au moins une cabine de balances (40) qui est disposée en tant que support de la réception de récipient (4) entre la réception de récipient (4) et le cadre (2).

4. Dispositif de récipient (1) selon une des revendications précédentes, dans lequel le cadre (2) présente sur son côté inférieur des roues (22) et/ou rouleaux au moyen desquelles le dispositif de récipient (1) peut être déplacé en tant qu'unité.

5. Dispositif de récipient (1) selon une des revendications précédentes, dans lequel la réception de récipient (4) présente une amenée d'agent d'équilibrage de température (44) et une évacuation d'agent d'équilibrage de température (46) qui peuvent être connectées à un dispositif de régulation d'équilibrage de température pour équilibrer la température du récipient reçu dans la réception de récipient (4).

6. Dispositif de récipient (1) selon une des revendications précédentes, dans lequel le dispositif de récipient (1) présente un dispositif d'agitation qui est disposé sur le cadre (2) et sous la réception de récipient (4).

7. Dispositif de récipient (1) selon une des revendications précédentes, dans lequel la soupape (6) est une soupape de serrage, et/ou dans lequel le dispositif de pompe (8) présente une pompe péristaltique (24, 26).

8. Dispositif de récipient (1) selon une des revendications précédentes, dans lequel la réception de récipient (4) présente une ouverture de passage (38) pour le passage de la conduite de fluide, dans lequel la soupape (6) est disposée sur le cadre (2) dans la région de l'ouverture de passage (38).

9. Dispositif de récipient (1) selon une des revendications précédentes, dans lequel le dispositif de commande (10) présente un système de bus pour la communication avec un dispositif externe.

10. Dispositif de récipient (1) selon une des revendications précédentes, dans lequel le dispositif de commande (10) présente un dispositif de saisie.
